# EUROPEAN PATENT APPLICATION

(11) **EP 4 122 442 A1**
(43) Date of publication of application: **25.01.2023**
(21) Application number: 21862119.1
(22) Date of filing: 27.08.2021
(51) Int. Cl.: A61K 8/85, A61K 8/90, A61K 8/84, A61K 8/73, A61K 8/04, A61Q 19/00, A61K 9/10, A61K 8/02, A61K 31/765

(54) **BIODEGRADABLE POLYMER DISPERSION, COMPOSITION COMPRISING SAME, AND SKIN IMPROVEMENT SYSTEM**

(30) Priority: 31.08.2020 KR 20200110235; 26.08.2021 KR 20210112934
(71) Applicant: Vaim Co. Ltd., Okcheon-gun, Chungcheongbuk-do 29055 (KR)
(72) Inventor: KIM, Gun Poong, Nonsan-si Chungcheongnam-do 33027 (KR); SEO, Suk Bae, Seoul 06732 (KR); LEE, Kun Pil, Daejeon 34379 (KR)
(74) Representative: Bassil, Nicholas Charles
(86) International application number: PCT/KR2021/011507
(87) International publication number: WO 2022/045823

(57) **Abstract**

The present disclosure relates to a biodegradable polymer dispersion containing a lactic acid-based polymer, a lactic acid-based polymer and polyethylene glycol-containing block copolymer (PEG-PLA), and a hyaluronic acid mixture, wherein the biodegradable polymer dispersion has excellent physiological activity by itself, has excellent dispersion stability of the lactic acid-based polymer, and is capable of effectively supporting active ingredients.

In addition, the present disclosure relates to a composition containing the biodegradable polymer dispersion and a skin improvement system.

## Description

### [Technical Field]

The present disclosure relates to a biodegradable polymer dispersion, a composition comprising the same, and a skin improvement system.

### [Background Art]

The skin includes epidermis, dermis and subcutaneous tissue. The dermis is a layer between the epidermis and subcutaneous tissue, and contains blood vessels, collagen, elastin fibers, pores, arrector pili muscles, sebaceous glands, sweat glands, various sensory nerves, fibroblasts, and macrophages that are not present in the epidermis and occupy the largest portion of the skin. In order to improve skin condition through percutaneous absorption of active ingredients, it is necessary to penetrate a skin barrier layer existing in the epidermis. However, since most of the active ingredients do not penetrate the barrier layer and are not delivered to a deep tissue of the skin, in order to enhance the percutaneous absorption of the active ingredients, use of a chemical absorption accelerator, a method of physically forming micropores in the barrier layer, or a method of delivering the active ingredients via electrophoresis have been considered.

On the other hand, pharmaceutical approaches have been extensively studied to enhance penetration and permeation of the active ingredients that are difficult to absorb into the skin, and in particular, the development of drug delivery systems such as colloids or nanoparticles or formulation approaches have been intensively studied.

As an active ingredient delivery system based on the drug delivery system, nanoparticles such as liposomes, cationic polymers, quantum dots, magnetic particles, or gold nanoparticles have been studied, and these delivery systems are designed to promote absorption into cells. The liposome has the advantage of being easily designed to have targeting performance as a physical self-assembly, but has poor colloidal stability of the delivery system, and depends mainly on the delivery of the active ingredients by diffusion in the skin barrier layer. In addition, when a cationic polymer, quantum dots, gold nanoparticles, etc. are used, there are disadvantages in that some cytotoxicity exists, effective delivery of an effective material is not easy, and the delivery system does not have biodegradability.

In order to solve theses problems, recently, a system for delivering or controlling the release of active ingredients by encapsulating active ingredients in a carrier using a biodegradable polymer has been studied. However, the system is mainly suitable for encapsulating active ingredients having a small molecular weight, and when the active ingredients having a high molecular weight are encapsulated, it is difficult to control the release of the active ingredients, and there are problems such as an initial excessive release. Moreover, the system is used for subcutaneous and intramuscular injection, so it is not suitable for application as an external preparation for the skin.

Therefore, there is a need for an active ingredient delivery system suitable as an external preparation for the skin and capable of maximizing physiological activity in cells and efficiently delivering active ingredients.

### [Related Art Document]

### [Patent Document]

Korean Patent Laid-Open Publication No. 10-2019-0095088 (published on August 19, 2019)

### [Disclosure]

### [Technical Problem]

An object of the present disclosure is to provide a lactic acid-based polymer and a composition containing the same, specifically, a biodegradable polymer dispersion containing a lactic acid-based polymer and polyethylene glycol-containing block copolymer (PEG-PLA) positioned on a surface of lactic acid-based polymer particles and a hyaluronic acid mixture, and having physiological activity by penetrating into the skin itself. Another object of the present disclosure is to provide a composition containing a biodegradable polymer dispersion capable of effectively delivering active ingredients into the deep of the skin by including the active ingredients inside the lactic acid-based polymer particles.

Another object of the present disclosure is to provide a skin improvement system including the biodegradable polymer dispersion and a plasma device so as to enhance skin absorption of active ingredients.

### [Technical Solution]

In one general aspect, there is provided a biodegradable polymer dispersion containing a lactic acid-based polymer, a lactic acid-based polymer and polyethylene glycol-containing block copolymer, and a hyaluronic acid mixture.

The biodegradable polymer dispersion may be spherical particles having an average particle size of 0.01 to 30 um.

The lactic acid-based polymer may have a weight average molecular weight of 10,000 to 1,000,000 g/mol.

The lactic acid-based polymer and polyethylene glycol-containing block copolymer may have a weight average molecular weight of 2,000 to 60,000 g/mol.

A weight ratio of the lactic acid-based polymer to the lactic acid-based polymer and polyethylene glycol-containing block copolymer may be 1:1 to 20:1.

The hyaluronic acid mixture may include hyaluronic acid, ammonium-substituted hyaluronic acid, and a hyaluronic acid oligomer having a weight average molecular weight of less than 8,000 g/mol.

The high molecular weight hyaluronic acid may be contained in an amount of 50 to 90% by weight, the ammonium-substituted hyaluronic acid may be contained in an amount of 1 to 10% by weight, and the hyaluronic acid oligomer may be contained in an amount of 10 to 35% by weight, based on the total weight of the hyaluronic acid mixture.

A weight ratio of the hyaluronic acid mixture to the lactic acid-based polymer may be 1:3 to 1:20.

The lactic acid-based polymer and polyethylene glycol-containing block copolymer (PEG-PLA) may be positioned on the surface of the particles including the lactic acid-based polymer.

In another general aspect, there is provided a cosmetic composition containing the biodegradable polymer dispersion.

In another general aspect, there is provided a composition for an external preparation for the skin containing the biodegradable polymer dispersion as described above.

In another general aspect, there is provided a pharmaceutical composition containing the biodegradable polymer dispersion as described above.

The biodegradable polymer dispersion may include active ingredients inside the particles including the lactic acid-based polymer.

In another general aspect, there is provided a skin improvement system including the biodegradable polymer dispersion as described above and a plasma device.

### [Advantageous Effects]

The biodegradable polymer dispersion according to the present disclosure contains a lactic acid-based polymer in the form of particles in which the lactic acid-based polymer and polyethylene glycol-containing block copolymer (PEG-PLA) are positioned on a surface in a dispersed form in an aqueous phase containing a hyaluronic acid mixture, and thus, has excellent dispersion stability, may control a particle size of the lactic acid-based polymer, and has physiological activity by penetrating into the skin itself.

In addition, the biodegradable polymer dispersion according to the present disclosure contains the active ingredients, such that the active ingredients may be effectively delivered through the skin or other barrier. Specifically, the active ingredients may include both hydrophilic and hydrophobic active ingredients. Thus, there is an advantage in that excellent physiological activity and effects of treatment or improvement may be implemented. Furthermore, active ingredients may be further contained in the lactic acid-based polymer particles of the biodegradable polymer dispersion to control continuous release of the active ingredients.

The biodegradable polymer dispersion according to the present disclosure has an excellent skin improvement effect by further improving the physiological activity effect and skin penetration by the plasma device.

### [Description of Drawings]

FIG. 1 is a schematic diagram illustrating a biodegradable polymer dispersion according to Example 1 of the present disclosure.
FIGS. 2 to 18 illustrate confocal microscopy and scanning electron microscope (SEM) photographs of biodegradable polymer dispersions according to Examples and Comparative Examples of the present disclosure.
FIG. 19 is a graph illustrating a result of confirming the cytotoxicity of the biodegradable polymer dispersions according to Examples 1 and 4 of the present disclosure in human dermal fibroblasts.
FIG. 20 is a graph obtained by evaluating a procollagen synthesis ability before and after plasma treatment in human dermal fibroblasts of the biodegradable polymer dispersions according to Examples 1 and 4 of the present disclosure.
FIG. 21 is a graph obtained by evaluating an MMP-1 inhibitory ability before and after plasma treatment in human dermal fibroblasts of the biodegradable polymer dispersions according to Examples 1 and 4 of the present disclosure.
FIG. 22 is a graph obtained by evaluating an elastase inhibition rate before and after plasma treatment in human dermal fibroblasts of the biodegradable polymer dispersions according to Examples 1 and 4 of the present disclosure.
FIG. 23A is an image illustrating an amount of collagen expressed before and after plasma treatment in human dermal fibroblasts of the biodegradable polymer dispersions according to Examples 1 and 4 of the present disclosure.
FIG. 23B is an image illustrating an amount of elastin expressed before and after plasma treatment in human dermal fibroblasts of the biodegradable polymer dispersions according to Examples 1 and 4 of the present disclosure.

### [Best Mode]

Hereinafter, the present disclosure will be described in more detail with reference to embodiments and examples including accompanying drawings. The following specific examples and embodiments are only a reference for describing the present disclosure in detail, and the present disclosure is not limited thereto, and may be implemented in various forms.

In addition, all technical terms and scientific terms have the same meanings as those commonly understood by a person skilled in the art to which the present disclosure pertains unless otherwise defined. The terms used in the description of the present disclosure are only for effectively describing certain embodiments, and are not intended to limit the present disclosure.

In addition, singular forms used in the detailed description and the claims are intended to include the plural forms unless otherwise indicated in context.

Unless explicitly described otherwise, "including" any component will be understood to imply the inclusion of other components rather than the exclusion of other components.

The term "lactic acid-based polymer particle" as used in the present disclosure has the same meaning as "particles containing lactic acid-based polymer."

The term "PEG-PLA block copolymer" as used in the present disclosure is used as a more specific meaning of "lactic acid-based polymer and polyethylene glycol-containing block copolymer."

The term "composition" as used in the present disclosure is used as the meaning including "cosmetic composition," "composition for an external preparation for the skin", or "pharmaceutical composition."

The term "dispersion" as used in the present disclosure means a "dispersion formed in an aqueous phase," and is used as a meaning including a "biodegradable polymer dispersion."

The present disclosure for achieving the above object provides a biodegradable polymer dispersion, a composition comprising the same, and a skin improvement system.

The present inventors have intensified research on a delivery system that has excellent physiological activity, may effectively improve or treat skin due to excellent penetration into the skin barrier, and has no toxicity in vivo. Thus, the present inventors have completed the present disclosure by confirming that the above effects may be implemented through a biodegradable polymer dispersion containing a lactic acid-based polymer, the lactic acid-based polymer and polyethylene glycol-containing block copolymer (PEG-PLA block copolymer), and a hyaluronic acid mixture.

Hereinafter, the biodegradable polymer dispersion according to the present disclosure will be described in detail.

The biodegradable polymer dispersion according to an embodiment of the present disclosure may include a lactic acid-based polymer, a lactic acid-based polymer and polyethylene glycol-containing block copolymer, and a hyaluronic acid mixture.

The lactic acid-based polymer means a polymer including lactic acid as a structural unit, and the lactic acid may be L-lactic acid, D-lactic acid, or a combination thereof. The lactic acid unit may be included in an amount of 50 mol% or more, specifically 60 mol% or more, and more specifically 70 mol% or more, based on 100 mol% of all monomer components constituting the lactic acid-based polymer, but the present disclosure is not limited thereto. The lactic acid-based polymer may include poly(lactic acid) (PLA), poly(lactic-co-glycolic acid(PLGA), etc., preferably poly(lactic acid) (PLA).

The lactic acid-based polymer may have a weight average molecular weight of 10,000 to 1,000,000 g/mol, specifically 13,000 to 500,000 g/mol, and more specifically, 15,000 to 250,000 g/mol.

The lactic acid-based polymer and polyethylene glycol-containing block copolymer may mean a polymer including a lactic acid-based polymer and a polyethylene glycol unit, and specifically a double block copolymer of a lactic acid-based polymer and polyethylene glycol (PEG-PLA block copolymer). In this case, the lactic acid-based polymer may have hydrophobic properties, and polyethylene glycol may have hydrophilic properties, and thus may have amphiphilic properties.

In the lactic acid-based polymer and polyethylene glycol-containing block copolymer, the lactic acid-based polymer may be a polymer of a monomer selected from the group consisting of L-lactic acid, D-lactic acid and L,D-lactic acid, and may have a weight average molecular weight of 1,000 to 40,000 g/mol, and specifically 1,500 to 30,000 g/mol. The block copolymer may have a weight average molecular weight of 2,000 to 60,000 g/mol, and polyethylene glycol in the block copolymer may have a weight average molecular weight of 1,000 to 20,000 g/mol, and specifically 3,000 to 15,000 g/mol, but the present disclosure is not limited thereto. In the block copolymer, a weight ratio of the lactic acid-based polymer block and the polyethylene glycol block may be 95:5 to 50:50, specifically 90:10 to 70:30, and more specifically 90:10 to 80:20.

The lactic acid-based polymer, and the lactic acid-based polymer and polyethylene glycol-containing block copolymer may be included in a weight ratio of 1:1 to 20:1. The lactic acid-based polymer and the block copolymer in the above weight ratio range are included together, such that particle size controllability and dispersion stability of the lactic acid-based polymer particles of the present disclosure may be improved, and miscibility with a hyaluronic acid mixture to be described later may be improved, which may be more preferable. In addition, a weight ratio of the lactic acid-based polymer and the block copolymer may be adjusted to control a size and a shape of the dispersion formed in the aqueous phase.

More specifically, if the weight ratio of the lactic acid-based polymer to the block copolymer is 1:1 to 6:1, the dispersion may be made of fine particles having a size of 0.01 to 4 um, and the inside of the fine particles will have a spherical shape filled with a high density of lactic acid-based polymer.

If the weight ratio of the lactic acid-based polymer to the block copolymer is 6:1 to 9:1, the dispersion may be prepared as spherical or elliptical particles elongated in one direction. If the weight ratio is 9:1 to 15:1, the dispersion may have a spherical or elliptical shape in which pores are formed therein.

The dispersion formed in the aqueous phase by including the lactic acid-based polymer, and the lactic acid-based polymer and polyethylene glycol-containing block copolymer may be spherical or elliptical particles, and the particles may have an average particle size of 0.01 to 30 um, specifically, 0.1 to 20 um.

In the case of a dispersion in which the lactic acid-based polymer is used alone, the average particle size may be atomized depending on a preparing process, but there is a problem in that a polydispersity is high and a reproducibility of the dispersion is poor. In contrast, the biodegradable polymer dispersion according to the present disclosure contains the lactic acid-based polymer and polyethylene glycol-containing block copolymer. Thus, the dispersion has advantages of very high reproducibility depending on a preparing method, enabling atomizing even when high energy is not applied, and having high dispersity of particles.

The hyaluronic acid mixture may mean a hyaluronic acid mixture of three types of hyaluronic acid, ammonium-substituted hyaluronic acid, and hyaluronic acid oligomer.

In the hyaluronic acid mixture, the hyaluronic acid may be a high molecular weight hyaluronic acid having a weight average molecular weight of 1,000,000 g/mol to 3,000,000 g/mol, specifically, 1,200,000 g/mol to 2,200,000 g/mol, and may be included in an amount of 55 to 90% by weight, and specifically 65 to 85% by weight, based on the total weight of the hyaluronic acid mixture, but the present disclosure is not limited thereto.

The ammonium-substituted hyaluronic acid may mean that some or all of the hydrogen atoms of the hydroxyl group of the hyaluronic acid are substituted with a group having a quaternary ammonium cation group, may have a weight average molecular weight of 300,000 g/mol to 1,000,000 g/mol, and specifically 500,000 g/mol to 800,000 g/mol, and may be included in an amount of 1 to 10% by weight, and specifically 3 to 8% by weight, based on the total weight of the hyaluronic acid mixture, but the present disclosure is not limited thereto.

The hyaluronic acid oligomer may refer to a hydrolyzed state of hyaluronic acid, and may refer to a low molecular weight hyaluronic acid having a weight average molecular weight of less than 8,000 g/mol, specifically less than 5,000 g/mol. A lower limit of the weight average molecular weight thereof is not limited, but may be 800 g/mol or more. The hyaluronic acid oligomer may be included in an amount of 10 to 35% by weight, and specifically 13 to 30% by weight, based on the total weight of the hyaluronic acid mixture, but the present disclosure is not limited thereto.

The weight ratio of the hyaluronic acid mixture to the lactic acid polymer may be 1:3 to 1:20, specifically 1:4 to 1:16, but the present disclosure is not limited thereto.

A mixture of three different types of hyaluronic acid as described above may be used to further improve the dispersion stability of the lactic acid-based polymer particles of the biodegradable polymer dispersion to be described later. In particular, the hyaluronic acid mixture may be combined with the lactic acid-based polymer particles to significantly improve the interaction with the skin barrier layer. It is known that conventional lactic acid-based polymers have high crystallinity and low interaction with a lipid layer constituting the skin barrier layer, so that their adsorption or permeation properties to the skin barrier layer are significantly low. However, the hyaluronic acid mixture is mixed with the lactic acid-based polymer particles to hydrate the surface of the lactic acid-based polymer particles. Thus, the interaction of the lactic acid-based polymer particles with the skin barrier layer is significantly improved, and thus may be strongly adsorbed or permeated to the skin barrier layer. Accordingly, when the biodegradable polymer dispersion is applied to the skin, physiological activity such as improving elasticity of skin cell, inhibiting aging, and promoting growth of skin cells may be further improved, which is preferable.

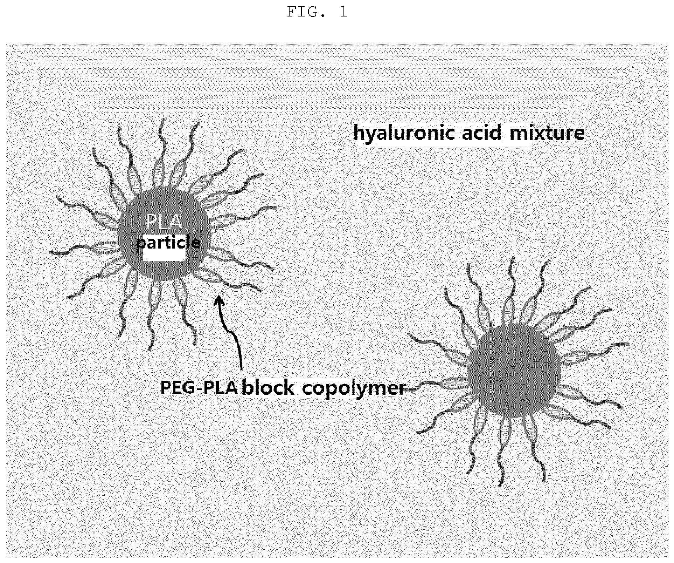

As illustrated in FIG. 1, in the biodegradable polymer dispersion according to a preferred embodiment of the present disclosure, lactic acid-based polymer particles may be dispersed on a continuous phase including a hyaluronic acid mixture, and a lactic acid-based polymer and polyethylene glycol-containing block copolymer may be positioned on the surface of the lactic acid-based polymer particles. More specifically, in the block copolymer, the hydrophobic lactic acid-based polymer portion is positioned to face the lactic acid-based polymer particles, and hydrophilic polyethylene glycol is positioned to face the continuous phase. At this time, by the hyaluronic acid mixture in which three different hyaluronic acids are mixed to form a continuous phase, the lactic acid-based polymer portion, which is the hydrophobic portion of the block copolymer, may be more densely located on the surface of the particle, and excellent dispersion stability may be implemented by polyethylene glycol, which is a hydrophilic portion, such that a large amount of lactic acid-based polymer particles as a dispersed phase ma be accommodated in the continuous phase, which may be more preferable. Accordingly, the biodegradable polymer dispersion may include active ingredients to be described later in each phase independently, which may be more preferable.

Hereinafter, a preparing method of the biodegradable polymer dispersion according to the present disclosure will be described in detail.

The production method of a biodegradable polymer dispersion according to the present invention may include: preparing a polymer solution by dissolving the polylactic acid-based polymer and the block copolymer in a solvent; preparing an aqueous solution in which a hyaluronic acid mixture and water are mixed; preparing a dispersion liquid by mixing the aqueous solution with the polymer solution; and removing the solvent from the dispersion liquid.

The preparing of the polymer solution may include dissolving the above-described polylactic acid-based polymer and block copolymer in a solvent. The solvent is not limited as long as it is an organic solvent miscible with water, and may be, specifically, any one or a mixture of two or more selected from the group consisting of acetone, ethanol, acetic acid, N,N-dimethylformamide, and N,N-dimethylacetamide, preferably acetone.

The solvent may be included in an amount of 70% to 95% by weight, based on the total weight of the polymer solution, but the present invention is not limited thereto.

In the preparing of the aqueous solution, the three types of hyaluronic acid described above may be used, and the water may be included in an amount of 95% to 99.9% by weight, based on the total aqueous solution, but the present invention is not limited thereto.

The preparing of the dispersion liquid may include mixing the aqueous solution with the polymer solution, and the polymer solution and the aqueous solution may be prepared by mixing in a volume ratio of 1:1 to 1:3. In this case, the dispersion liquid may be one in which an aqueous solution forms a continuous phase, a solvent is extracted into the continuous phase, and a phase-separated polymer forms a dispersed phase. Specifically, the dispersed phase may be a lactic acid-based polymer and polyethylene glycol-containing block copolymer positioned on the surface of the dispersed lactic acid-based polymer particles.

The removing of the solvent is not limited, but may specifically use evaporation, and the evaporation condition may be performed at 20 to 130°C for 1 hour to 48 hours, but the present disclosure is not limited thereto. Depending on the type of solvent to be used, evaporation conditions may be adjusted. In addition, the evaporation may be performed under reduced pressure.

The biodegradable polymer dispersion of the present disclosure prepared through the above steps may be in a form in which the lactic acid-based polymer particles are dispersed on a continuous phase including the hyaluronic acid mixture, and the lactic acid-based polymer particles have a lactic acid-based polymer and polyethylene glycol-containing block copolymer positioned on the surface.

The biodegradable polymer dispersion according to an aspect of the present disclosure itself has excellent physiological activity properties, biocompatibility and high supporting properties that may contain a large amount of active ingredients and may be applied to various fields, and specifically, a cosmetic composition or a composition for an external preparation for the skin, or a pharmaceutical composition containing the biodegradable polymer dispersion may be mentioned. More specifically, the application aspect of the active ingredients may include: a first aspect including hydrophilic active ingredients in a hyaluronic acid mixture, which is a hydrophilic continuous phase, a second aspect including hydrophobic active ingredients in a hydrophobic lactic acid-based polymer; and a third aspect including active ingredients in each of a hyaluronic acid mixture, which is a hydrophilic continuous phase, and the hydrophobic lactic acid-based polymer.

The active ingredients may be used without limitation as long as it is known active ingredients having physiological activity in the skin or other tissues, and may further include conventional additives that are not harmful to the human body, such as additional flavorings, vitamins, stabilizers, antioxidants, etc., within the range that does not impair the physical properties of the biodegradable polymer dispersion of the present disclosure, but the present disclosure is not limited thereto.

The biodegradable polymer dispersion according to the present disclosure described above or a composition containing the biodegradable polymer dispersion may be applied in formulations such as known internal and external preparations, and preferably may be applied as external preparations such as creams, ointments, lotions, and gels.

The biodegradable polymer dispersion or a composition containing the biodegradable polymer dispersion may be applied directly to the skin, and when plasma-treated to the applied site, the penetration of the biodegradable polymer dispersion into the skin may be increased and absorption into the skin may be promoted, so that the physiologically active properties may be accelerated. Therefore, it is possible to effectively improve the skin in a short time and to help the treatment by skin regeneration, and thus, it may be applied for skin improvement. In addition, when additional active ingredients are contained in the biodegradable polymer dispersion, the plasma treatment promotes the diffusion of the active ingredients into the deep skin, such that the skin improvement or therapeutic effect may be further maximized, which is preferable.

The biodegradable polymer dispersion according to an aspect of the present disclosure having the above properties or a composition containing the same may be provided with a plasma treatment device and used as a desirable skin improvement system.

Hereinafter, the present disclosure will be described in more detail on the basis of Examples and Comparative Examples. However, the following Examples and Comparative Examples are only examples for describing the present disclosure in more detail, and the present disclosure is not limited by the following Examples and Comparative Examples.

### [Experimental method]

### 1. Confirmation of production of polymer dispersion

The prepared biodegradable polymer dispersion was analyzed for the formation of the biodegradable polymer dispersion by confocal laser scanning microscopy (CLSM).

### 2. Measurement of physical properties of polymer dispersion

The prepared biodegradable polymer dispersion was freeze-dried, and a scanning electron microscope (SEM) photograph was measured, and the particle size and particle size distribution of the prepared biodegradable polymer were measured using a nano zeta potentiometer (Nano ZSP manufactured by Malvern Instruments Zetasizer).

### [Example 1]

A polymer solution was prepared in which 1.25 g of polylactic acid (EVONIK, RESOMER^{®} R 202 S) and 1.25 g of a PEG-PLA block copolymer (EBONIK, RESOMER 100 DL mPEG5000) were mixed in 25 mL of acetone.

6 g of hyaluronic acid (molecular weight 1,200,000 g/mol), 0.4 g of hydroxypropyltriammonium hyaluronic acid (molecular weight 500,000 g/mol), which is ammonium-substituted hyaluronic acid, and 1.6 g of sodium hyaluronate (molecular weight 5,000 g/mol) hydrolyzed with hyaluronic acid oligomer in 1,000 mL of distilled water were mixed to prepare an aqueous solution.

Then, a biodegradable polymer dispersion was prepared by slowly mixing 25 mL of the prepared polymer solution into 50 mL of an aqueous solution under stirring, and evaporating the mixed solution in acetone under reduced pressure at room temperature.

### [Example 2]

A biodegradable polymer dispersion was prepared in the same manner as in Example 1, except that 1.67 g of polylactic acid and 0.83 g of PEG-PLA block copolymer were used.

### [Example 3]

A biodegradable polymer dispersion was prepared in the same manner as in Example 1, except that 1.87 g of polylactic acid and 0.63 g of PEG-PLA block copolymer were used.

### [Example 4]

A biodegradable polymer dispersion was prepared in the same manner as in Example 1, except that 2.08 g of polylactic acid and 0.5 g of PEG-PLA block copolymer were used.

### [Example 5]

A biodegradable polymer dispersion was prepared in the same manner as in Example 1, except that 2.08 g of polylactic acid and 0.42 g of PEG-PLA block copolymer were used.

### [Example 5]

A biodegradable polymer dispersion was prepared in the same manner as in Example 1, except that 2.08 g of polylactic acid and 0.42 g of PEG-PLA block copolymer were used.

### [Example 6]

A biodegradable polymer dispersion was prepared in the same manner as in Example 1, except that 2.14 g of polylactic acid and 0.36 g of PEG-PLA block copolymer were used.

### [Example 7]

A biodegradable polymer dispersion was prepared in the same manner as in Example 1, except that 2.22 g of polylactic acid and 0.28 g of PEG-PLA block copolymer were used.

### [Example 8]

A biodegradable polymer dispersion was prepared in the same manner as in Example 1, except that a solution obtained by mixing 3.0 g of polylactic acid and 0.3 g of PEG-PLA block copolymer in 33 mL of acetone was used.

### [Example 9]

A biodegradable polymer dispersion was prepared in the same manner as in Example 1, except that a solution obtained by mixing 4.5 g of polylactic acid and 0.3 g of PEG-PLA block copolymer in 48 mL of acetone was used.

### [Example 10]

A biodegradable polymer dispersion was prepared in the same manner as in Example 1, except that a solution obtained by mixing 6.0 g of polylactic acid and 0.3 g of PEG-PLA block copolymer in 63 mL of acetone was used.

### [Example 11]

A biodegradable polymer dispersion was prepared in the same manner as in Example 1, except that 1.5 g of polylactate-co-glyclate (EVONIK, RESOMER RG 752 S) was used instead of polylactic acid and 0.5 g of PEG-PLA copolymer was used.

### [Comparative Example 1]

A biodegradable polymer dispersion was prepared in the same manner as in Example 1, except that 50 mL of water (H₂O) was used instead of the aqueous solution.

### [Comparative Example 2]

A biodegradable polymer dispersion was prepared in the same manner as in Example 1, except that 50 mL of water (H₂O) was used instead of the aqueous solution in Example 2.

### [Comparative Example 3]

A biodegradable polymer dispersion was prepared in the same manner as in Example 1, except that 50 mL of water (H₂O) was used instead of the aqueous solution in Example 4.

### [Comparative Example 4]

A biodegradable polymer dispersion was prepared in the same manner as in Example 1, except that 50 mL of water (H₂O) was used instead of the aqueous solution in Example 7.

### [Comparative Example 5]

A biodegradable polymer dispersion was prepared in the same manner as in Example 1, except that the polylactic acid was not used.

### [Comparative Example 6]

A biodegradable polymer dispersion was prepared in the same manner as in Example 1, except that the PEG-PLA block copolymer was not used.

The properties of the Examples and Comparative Examples were evaluated as follows.

### [Experimental Example 1] Generation and particle size evaluation of biodegradable polymer dispersion

The average particle sizes of the dispersed particles in the biodegradable polymer dispersions prepared in Examples 1 to 11 and Comparative Examples 1 to 6 were measured and shown in Table 1 below.

**[Table 1]**

| | Examples | | | | | | | | | | |
|---|---|---|---|---|---|---|---|---|---|---|---|
| | 1 | 2 | 3 | 4 | 5 | 6 | 7 | 8 | 9 | 10 | 11 |
| Average particle size (µm) | 0.5 | 2.3 | 2.5 | 3.6 | 4.2 | 4.5 | 5.4 | 6.0 | 8.5 | 2.0 | 2.5 |

| | Comparative Examples | | | | | | | | | | |
|---|---|---|---|---|---|---|---|---|---|---|---|
| Average particle size (µm) | 1 | 2 | 3 | 4 | 5 | 6 | | | | | |
| | 18 | 23 | 34 | 40 | 57 | 100 | | | | | |

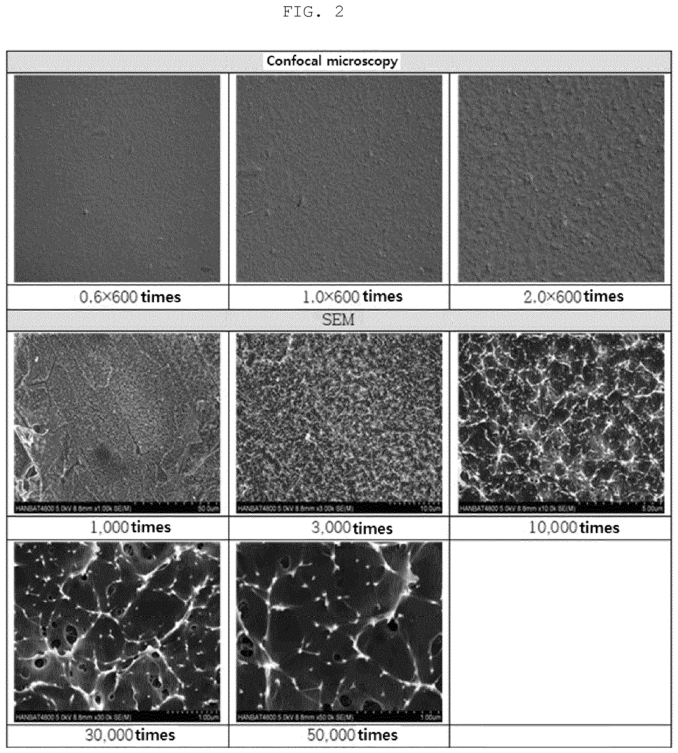

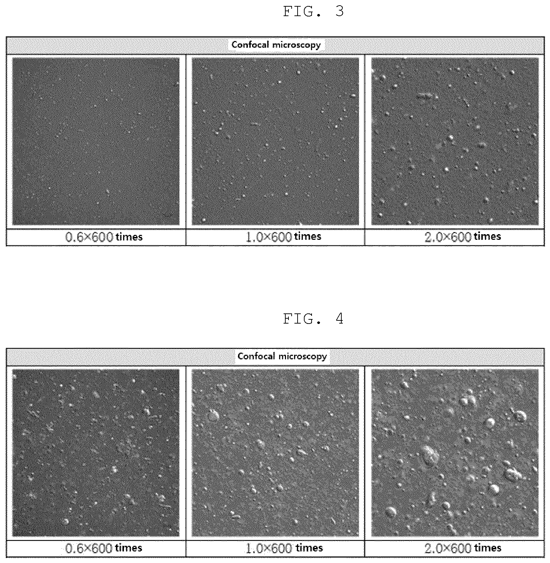

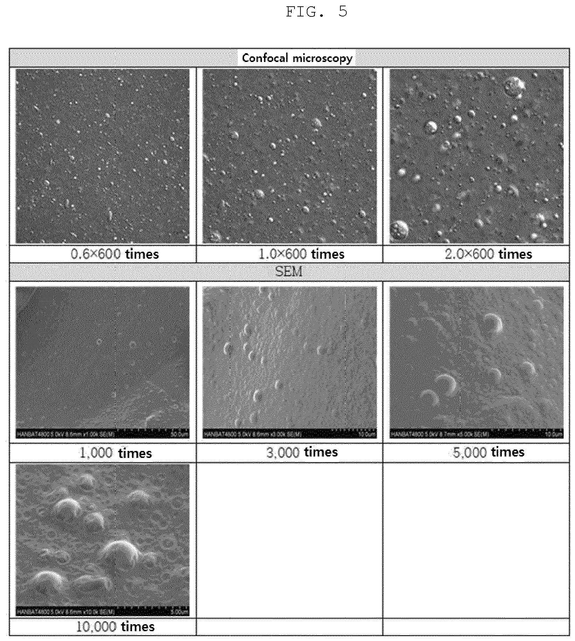

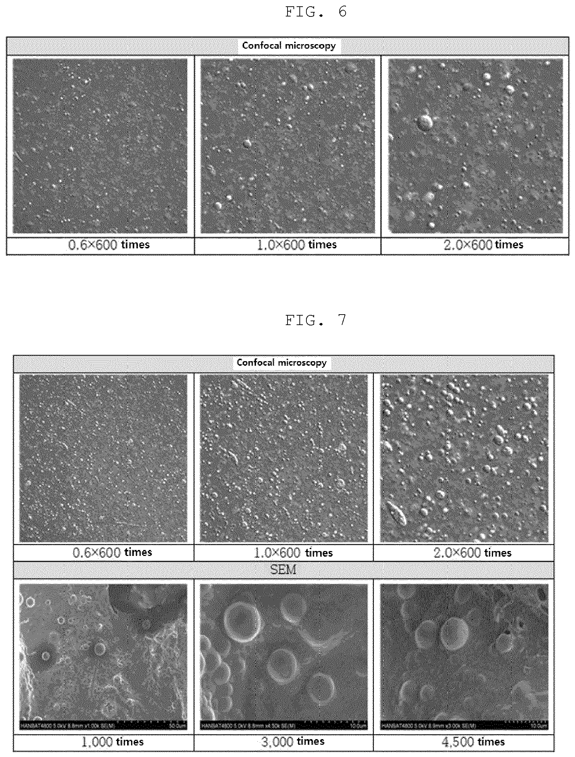

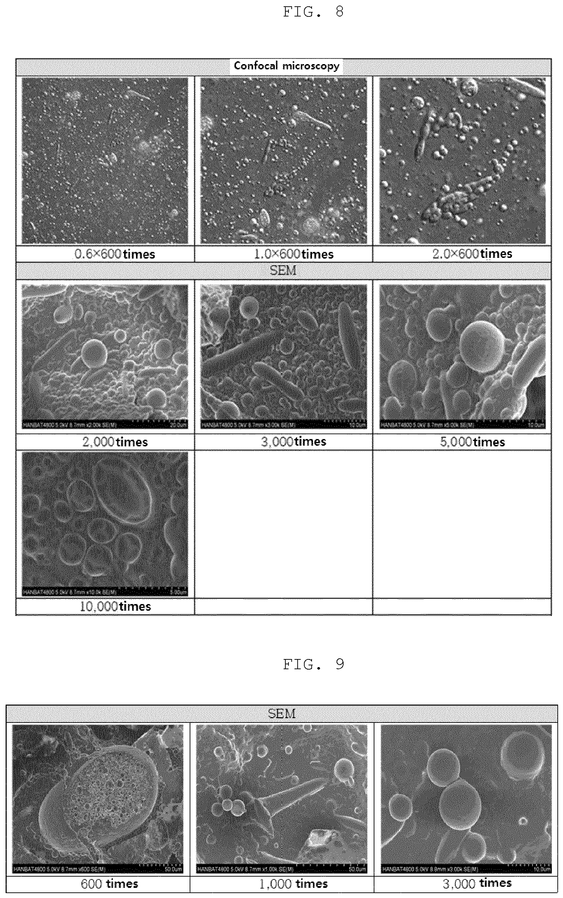

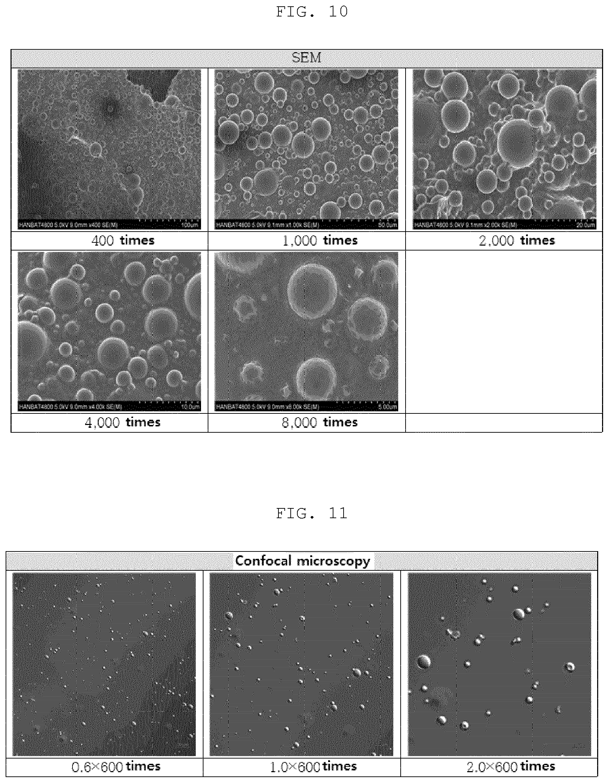

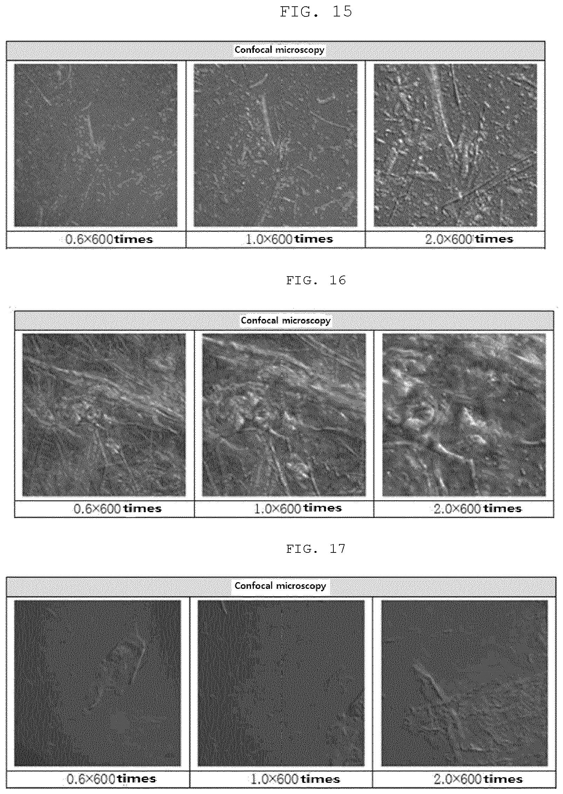

Confocal microscopy and scanning electron microscope (SEM) photographs of the biodegradable polymer dispersions prepared in Examples 1 to 11 and Comparative Examples 1 to 6 are illustrated in FIGS. 2 to 18, respectively.

As shown in Table 1 and FIGS. 2 to 12, in Examples 1 to 11, it could be confirmed that the biodegradable dispersion was formed into particles having a spherical or oval shape, and the average particle size had a size of less than 10 um.

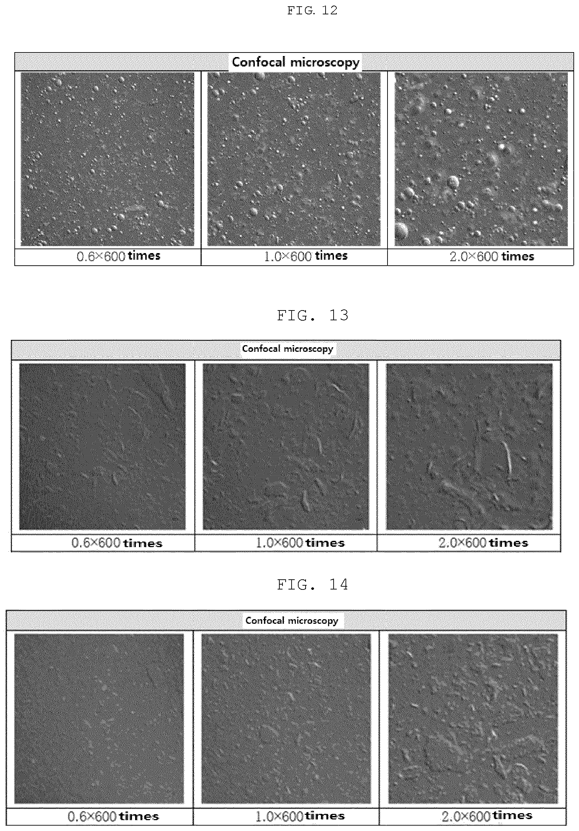

On the other hand, as in Table 1 and FIGS. 13 to 18, in Comparative Examples 1 to 4, it could be confirmed that when water was used instead of the aqueous solution in which three types of hyaluronic acid were mixed, the particles of the resulting biodegradable polymer dispersion were non-uniform and had an average particle size of 15 um or more. In addition, in Comparative Example 5, it could be confirmed that when polylactic acid (PLA) was not used, the particles of the resulting biodegradable polymer dispersion were largely agglomerated. Also, in Comparative Example 6 in which the PEG-PLA block copolymer was not used, it could be confirmed that polylactic acid (PLA) was largely agglomerated, resulting in the formation of precipitates rather than fine particles.

### [Experimental Example 2] Evaluation of physiological activity properties of biodegradable polymer dispersion

Changes in physiological activity before and after plasma irradiation were measured for the biodegradable polymer dispersions prepared according to Examples 1 and 4 above. A helium plasma irradiation device was used for plasma, and it was carried out according to the following conditions.

Human dermal fibroblasts (HDF-N) (GIBCO) were cultured in a medium containing Medium 106 (Gibco-BRL, Gaithersburg, MD) medium and a low-serum growth supplement (LSGS; Gibco-BRL, Gaithersburg, MD) and supplemented with 1% penicillin/streptomycin at 37°C and 5% CO₂ conditions.

**[Table 2]**

| | |
|---|---|
| Input Voltage | 12VDC |
| Output Voltage | ±2.5kV |
| Frequency | 10-15 kHz |
| Plasma Density | 10¹¹~10¹²/cm³ |

### 2-1. Cytotoxicity Assessment

Human dermal fibroblasts (HDF-N) were plated into a 96-well plate, and cultured at 37°C and 5% CO₂ conditions for 24 hours. Thereafter, the medium was replaced with a medium from which the supplement has been removed and cultured for 24 hours. In the case of a positive control, the test substance was diluted and replaced with the medium, and cultured for 24 hours. Thereafter, it was further cultured for 4 hours in an incubator at 37°C by treating the MTT solution with a concentration of 5 mg/ml. After culturing, the supernatant was removed, and DMSO was added to the formazan formed by MTT reduction to lyse the cells, and then the absorbance was measured at 540 nm using an ELISA microplate reader (SoftMax Pro5, Molecular Devices, USA). All data are expressed as mean standard deviation. For statistical analysis of each data, a two-sided test of Student's t-test was performed, and it was determined that there was significance when p < 0.05 compared to the control group.

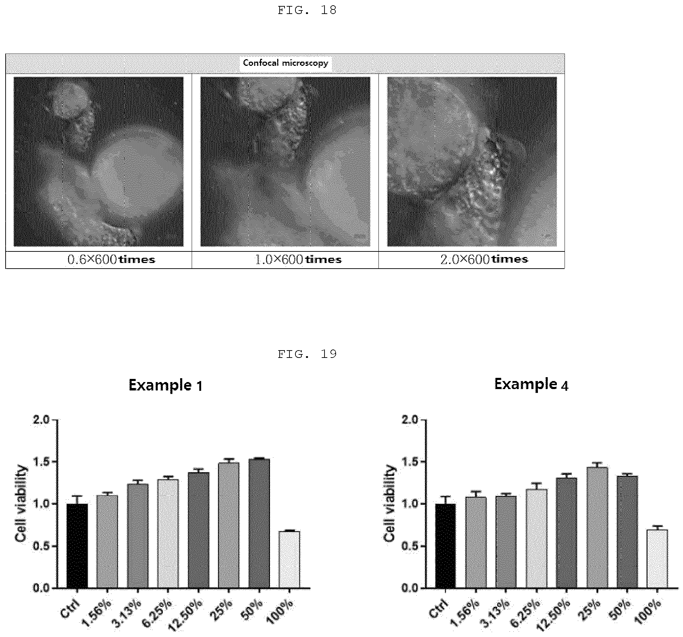

As a result of the experiment, as illustrated in FIG. 19, as a result of measuring the cell viability by diluting the biodegradable polymer dispersion according to specific Examples 1 and 4 of the present disclosure by concentration, it was analyzed that there was no cytotoxicity up to a concentration of 50%, and the stability was confirmed in human dermal fibroblasts.

### 2-2. Evaluation of procollagen synthesis ability

Human dermal fibroblasts (HDF-N) were plated into a 6-well plate, and cultured at 37°C and 5% CO₂ conditions for 24 hours. Thereafter, the medium was replaced with a medium from which the supplement has been removed, cells were kept starving for 24 hours, the biodegradable polymer dispersions according to Examples 1 and 4 were treated, and then plasma was irradiated for 3 seconds. After culturing for 24 hours, the medium of the cultured cells was collected and the amount of procollagen was measured using a procollagen Type I C-Peptide (PIP) ELISA kit (MK101, Takara Bio Inc.). All data are expressed as mean standard deviation. For statistical analysis of each data, a two-sided test of Student's t-test was performed, and it was determined that there was significance when p < 0.05 compared to the control group.

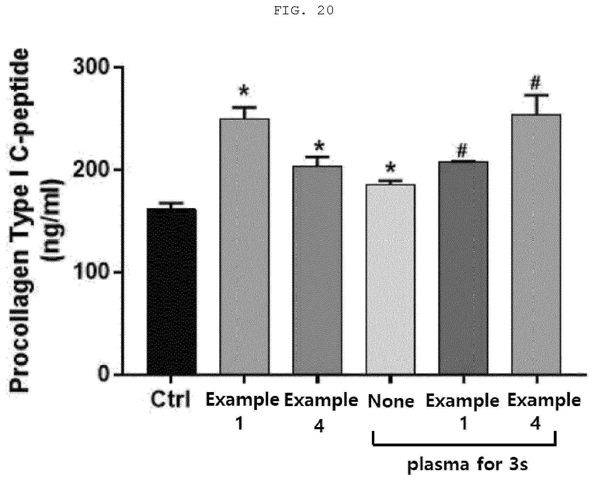

As a result of the experiment, as illustrated in FIG. 20, a significant effect was confirmed in Example 1, Example 4, and the group treated with only plasma, compared to the control group. In particular, it was found that in the group treated with plasma in the biodegradable polymer dispersion according to Example 1 and Example 4, the procollagen synthesizing ability was excellent. It was confirmed that through the excellent procollagen synthesis ability, the biodegradable polymer dispersion and plasma treatment according to the present disclosure may increase collagen biosynthesis in cells to provide skin regeneration and anti-aging effects.

### 2-3. Evaluation of MMP-1 inhibitory ability

Human dermal fibroblasts (HDF-N) were plated into a 6-well plate, and cultured at 37°C and 5% CO₂ conditions for 24 hours. Thereafter, the medium was replaced with a medium from which the supplement has been removed, and cells were kept starving for 24 hours. Then, the medium was removed, washing was performed with DPBS, and 30 mJ of UVB was irradiated by adding 200 µl of DPBS. After UVB irradiation, the biodegradable polymer dispersion according to Examples 1 and 4 was treated, and plasma was irradiated for 3 seconds. After culturing for 24 hours, the medium of the cultured cells was collected and the amount of MMP-1 was measured using a Human MMP-1 ELISA kit (ELH-MMP-1, RayBio Inc.). All data are expressed as mean standard deviation. For statistical analysis of each data, a two-sided test of Student's t-test was performed, and it was determined that there was significance when p < 0.05 compared to the control group.

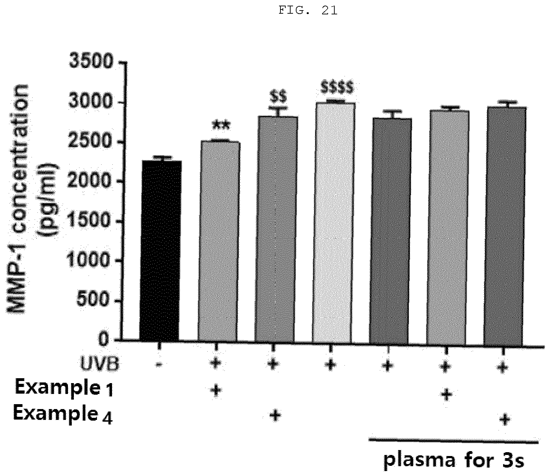

As a result of the experiment, as illustrated in FIG. 21, it was confirmed that the biodegradable polymer dispersion and plasma alone treatment group according to Examples 1 and 4 of the present disclosure significantly increased MMP-1, compared to the control group. However, in the case of the plasma treatment in Examples 1 and 4 in the inhibitory efficacy of MMP-1 expression, there was no significant difference.

### 2-4. Evaluation of elastase inhibition rate

Human dermal fibroblasts (HDF-N) were plated into a 6-well plate, and cultured at 37°C and 5% CO₂ conditions for 24 hours. Thereafter, the medium was replaced with a medium from which the supplement has been replaced, cells were kept starving for 24 hours, the biodegradable polymer dispersions according to Examples 1 and 4 were treated, and then plasma was irradiated for 3 seconds. The cultured cells were washed with DPBS, dissolved in 0.1% triton X-100 • 0.2M Tris solution (pH 8.0), and then frozen and thawed in liquid nitrogen three times to homogenize the cells. After the homogenized cells were centrifuged at 3000 rpm at 4°C for 20 minutes, the supernatant was taken to obtain an enzyme solution containing fibroblast elastase. The enzyme solution was quantified for protein, the same amount was taken in a volume of 98 µl in a 96-well plate, and 2 ul of N-succinyl-tri-alanyl-p-nitroanilide (STANA, 50 mM) solution, which is a substrate of elastase, was added to each well, and incubated at 37°C. After 90 minutes, the amount of elastase was measured with an ELISA Reader at 405 nm. All data are expressed as mean standard deviation. For statistical analysis of each data, a two-sided test of Student's t-test was performed, and it was determined that there was significance when p < 0.05, compared to the control group.

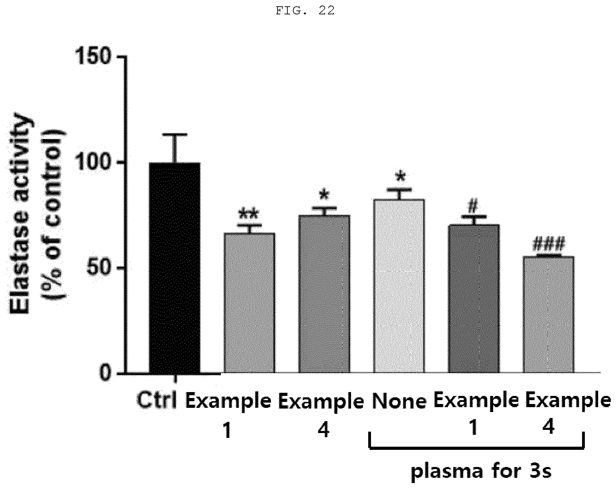

As a result of the experiment, as illustrated in FIG. 22, it was confirmed that the biodegradable polymer dispersion and plasma alone treatment group according to Examples 1 and 4 of the present disclosure significantly decreased the activity of elastase, compared to the control group. In particular, it could be confirmed that when plasma was treated with the biodegradable polymer dispersions according to Examples 1 and 4, the activity of elastase was further reduced, and the decomposition inhibitory effect of elastin was significantly exhibited.

### 2-5. Histological evaluation of artificial skin

The medium supplemented with FBS (Invitrogen-GIBCO-BRL, Grand Island, NY) was plated on a 12-well plate by at 1 ml, and artificial skin (Neoderm-ED, Tego Science) that reproduced the dermis and epidermis using human skin cells was carefully transferred and cultured at 37°C, 5% CO₂ culture condition for 24 hours. Thereafter, the biodegradable polymer dispersions prepared in Examples 1 and 4 were treated on artificial skin, and plasma was irradiated for 3 seconds. After culturing for 24 hours, the artificial skin was fixed in a 10% neutral buffered formalin (NBF) solution, then embedded in paraffin and hardened, and 5 um sections were prepared. And after MT staining (Masson Trichrome staining) and VVG staining (Verhoeff-van Gieson staining), the tissue of the artificial skin was observed with a tissue slide scanner (Panoramic MID II, 3D histech Ltd., HU).

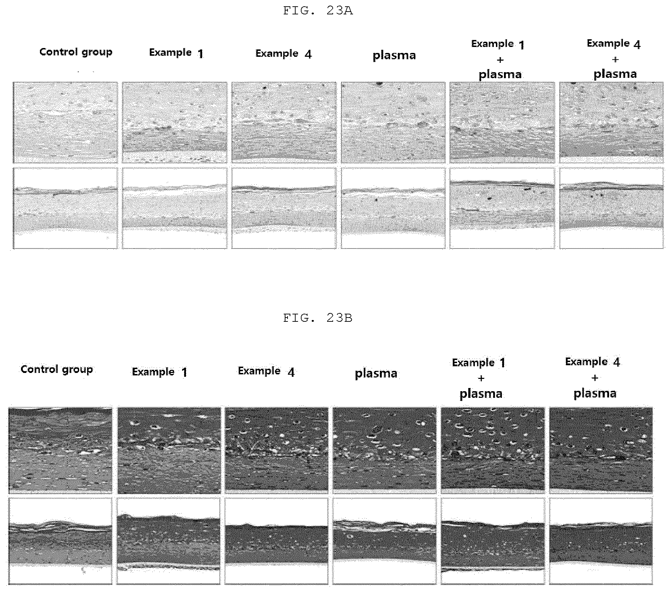

As a result of MT staining, as illustrated in FIG. 23A, it could be confirmed that in the biodegradable polymer dispersion and plasma alone treatment group according to Examples 1 and 4 of the present disclosure, the amount of collagen expressed was increased, compared to the control group. In particular, it could be confirmed that when the biodegradable polymer dispersion according to Examples 1 and 4 was treated with plasma, the amount of collagen expressed was further increased.

As a result of WG staining, as illustrated in FIG. 23B, it could be confirmed that in the biodegradable polymer dispersion and plasma alone treatment group according to Examples 1 and 4 of the present disclosure, the amount of elastin expressed was increased, compared to the control group. In particular, it could be confirmed that when the biodegradable polymer dispersion according to Examples 1 and 4 was treated with plasma, the amount of elastin expressed was further increased.

Hereinabove, although the present disclosure has been described by specific matters, the limited embodiments, and drawings, they have been provided only for assisting in a more general understanding of the present disclosure. Therefore, the present disclosure is not limited to the exemplary embodiments. Various modifications and changes may be made by those skilled in the art to which the present disclosure pertains from this description.

Therefore, the spirit of the present disclosure should not be limited to the above-mentioned embodiments, but the claims and all of the modifications equal or equivalent to the claims are intended to fall within the scope and spirit of the present disclosure.

## Claims

1. A biodegradable polymer dispersion comprising a lactic acid-based polymer, a lactic acid-based polymer and polyethylene glycol-containing block copolymer, and a hyaluronic acid mixture.

2. The biodegradable polymer dispersion of claim 1, wherein the biodegradable polymer dispersion is spherical particles having an average particle size of 0.01 to 30 um.

3. The biodegradable polymer dispersion of claim 1, wherein the lactic acid-based polymer has a weight average molecular weight of 10,000 to 1,000,000 g/mol.

4. The biodegradable polymer dispersion of claim 1, wherein the lactic acid-based polymer and polyethylene glycol-containing block copolymer has a weight average molecular weight of 2,000 to 60,000 g/mol.

5. The biodegradable polymer dispersion of claim 1, wherein a weight ratio of lactic acid-based polymer to the lactic acid-based polymer and polyethylene glycol-containing block copolymer is 1:1 to 20:1.

6. The biodegradable polymer dispersion of claim 1, wherein the hyaluronic acid mixture includes hyaluronic acid, ammonium-substituted hyaluronic acid, and a hyaluronic acid oligomer having a weight average molecular weight of less than 8,000 g/mol.

7. The biodegradable polymer dispersion of claim 6, wherein the high molecular weight hyaluronic acid is contained in an amount of 50 to 90% by weight, the ammonium-substituted hyaluronic acid is contained in an amount of 1 to 10% by wegiht, and the hyaluronic acid oligomer is contained in an amount of 10 to 35% by weight, based on the total weight of the hyaluronic acid mixture.

8. The biodegradable polymer dispersion of claim 1, wherein a weight ratio of the hyaluronic acid mixture to the lactic acid-based polymer is 1:3 to 1:20.

9. The biodegradable polymer dispersion of claim 2, wherein the lactic acid-based polymer and polyethylene glycol-containing block copolymer (PEG-PLA) is positioned on the surface of the particles including the lactic acid-based polymer.

10. A cosmetic composition comprising the biodegradable polymer dispersion of any one of claims 1 to 9.

11. A composition for an external preparation for the skin, comprising the biodegradable polymer dispersion of any one of claims 1 to 9.

12. A pharmaceutical composition, comprising the biodegradable polymer dispersion of any one of claims 1 to 9.

13. The pharmaceutical composition of claim 12, wherein the biodegradable polymer dispersion includes active ingredients inside the particles including the lactic acid-based polymer.

14. A skin improvement system, comprising the biodegradable polymer dispersion of any one of claims 1 to 9 and a plasma device.
